# EUROPÄISCHE PATENTSCHRIFT

(11) **EP 2 106 776 B1**
(45) Veröffentlichungstag und Bekanntmachung des Hinweises auf die Patenterteilung: **07.12.2011**
(21) Anmeldenummer: 08006233.4
(22) Anmeldetag: 31.03.2008
(51) Int. Cl.: A61F 13/56

(54) **Elastischer Verbundstoff**
Elastic adhesive material
Matière composite élastique

(43) Veröffentlichungstag der Anmeldung: 07.10.2009
(73) Patentinhaber: Nordenia Technologies GmbH, 48599 Gronau (DE)
(72) Erfinder: Baldauf, Georg, 48366 Laer (DE); Homölle, Dieter, 48607 Ochtrup (DE); Schönbeck, Marcus, 33775 versmold (DE)
(74) Vertreter: Albrecht, Rainer Harald

(56) Entgegenhaltungen:
- EP-A- 1 541 106
- WO-A-2006/008149
- JP-A- 9 075 394
- US-A1- 2006 257 666

## Beschreibung

Die Erfindung betrifft einen monoaxial dehnbaren elastischen Verbundstoff, insbesondere zur Fertigung von elastischen Seitenteilen an Windeln und elastischen Windelverschlussbändem, mit einem elastischen Träger, der in einer Richtung bevorzugt dehnbar ist und mit mindestens einer textilen Außenschicht, die aus einem Gewirke besteht. Um eine gute Passform der Windel sicherzustellen, müssen elastische Seitenteile an Windeln oder elastische Windelverschlussbänder sich durch eine hohe Elastizität auszeichnen und sich für die genannten Anwendungen um mehr als 20 mm dehnen lassen. Da sie mit dem Körper in Kontakt kommen können, sollen sie luftdurchlässig sein und eine weiche textile Oberfläche aufweisen. Schließlich wird für die genannten Anwendungen eine hohe Reißfestigkeit des elastischen Verbundstoffes gefordert.

Aus EP 0 839 222 B1 ist ein Verbundstoff bekannt, der eine erste textile Außenschicht aus einem Gewirke, eine zweite Außenschicht aus Spinnvlies und einen elastischen Träger als Kern aufweist. Der Träger besteht aus einzelnen elastischen Fäden, die sich in der bevorzugten Dehnungsrichtung des Verbundstoffes erstrecken. Die Fäden sind in einem geeigneten Bindemittel eingebettet, welches den Zwischenraum zwischen den Fäden ausfüllt und auch die textilen Außenschichten teilweise durchdringt. Das Material ist nicht luftdurchlässig und erfüllt damit ein wesentliches Kriterium für die vorgenannten Anwendungen nicht.

In WO 2004/094709 A2 wird ein Verbundstoff für Windeln beschrieben, der textile Außenschichten sowie einen aus zwei Schichten bestehenden elastischen Kern aufweist. Die Außenschichten können aus einem Gewirke bestehen. Der Kern weist ebenfalls eine Schicht aus einem Gewirke sowie elastische Fäden auf, die sich in der bevorzugten Dehnungsrichtung des Materials erstrecken. Die elastischen Fäden sind in einem geeigneten Bindemittel eingegossen, welches die Hohlräume zwischen den Außenschichten ausfüllt und die Außenschichten ebenfalls teilweise durchtränkt. Die textile Schicht des Kerns soll die Verbundfestigkeit bzw. die Haftung zwischen den einzelnen Schichten verbessern. Die elastischen Fäden sind in gedehntem Zustand in das Bindemittel eingegossen worden. Der Verbundstoff ist ebenfalls nicht luftdurchlässig.

Aus EP 0 233 364 B1 ist ein biaxial dehnbarer Verbundstoff mit wirktechnisch eingearbeiteten Schlaufen bekannt, der als Teil eines Klettverschlusses verwendet werden kann. Das Gewirke weist Filamente aus einem elastisch dehnbaren Material auf und ist auf einen elastischen Träger aufgebracht. Infolge seiner biaxialen Dehnbarkeit ist das Material nicht zur Fertigung von elastischen Seitenteilen an Windeln oder zur Fertigung von elastischen Windelverschlussbändern geeignet, denn für diese Anwendungen wird ein Verbundstoff gefordert, der monoaxial dehnbar ist und quer zur bevorzugten Dehnungsrichtung verhältnismäßig steif ist, so dass das Material bei einer Längendehnung nicht quer zur Dehnungsrichtung einschnürt.

Aus US 2006/0257666 A1 ist ein elastischer Verbundstoff bekannt, der einen elastischen Träger und mindestens eine aus einem Gewirke bestehende textile Außenschicht aufweist. Der elastische Träger besteht aus einem flächigen Material, welches nach einer monoaxialen Verstreckung eine bevorzugte Dehnungsrichtung aufweist und mit dem Gewirke verklebt ist. Der Druckschrift ist zu entnehmen, dass die textile Außenschicht ein Verblocken des Verbundstoffes verhindert, wenn das Material zu einer Rolle aufgewickelt wird. Vor diesem technologischen Hintergrund liegt der Erfindung die Aufgabe zugrunde, einen zur Fertigung von elastischen Seitenteilen an Windeln und elastischen Windelverschlussbändern geeigneten elastischen Verbundstoff anzugeben, der monoaxial dehnbar ist, sich durch gute Dehnungswerte und eine hohe Reißfestigkeit auszeichnet und eine weiche Textiloberfläche aufweist.

Die Aufgabe wird erfindungsgemäß durch einen Verbundstoff mit den Merkmalen des Anspruches 1 gelöst. Der elastische Träger des Verbundstoffes besteht aus einem flächigen Material, welches eine bevorzugte Dehnungsrichtung aufweist und mit dem Gewirke verklebt ist. Das Gewirke besteht aus einer Kettwirkware, die in der Dehnungsrichtung des Verbundstoffes ein hohes Dehnungsvermögen aufweist und in der Querrichtung dazu steif ist.

Das Gewirke verleiht dem erfindungsgemäßen Verbundstoff eine flauschige Oberfläche. Es zeichnet sich durch eine hohe Luftdurchlässigkeit und gute Dehnbarkeit aus. Der elastische Träger verleiht dem Verbundstoff die gewünschte Elastizität in der bevorzugten Dehnungsrichtung und bestimmt die für die beschriebenen Anwendungen notwendigen elastischen Rückstellkräfte. Vorzugsweise besteht der elastische Träger aus einem luftdurchlässigen Material. Für die Verklebung des Trägers und der textilen Außenschicht eignen sich prinzipiell alle auf dem Gebiet der Kaschierfolien verwendeten Klebstoffe. Bevorzugt sind Schmelzklebstoffe auf der Basis aliphatischer primärer Alphaolefine APAO, EVA, Styrol-Blockcopolymere wie SBS, SEBS oder SIS, reaktive Polyurethanklebstoffe, Acrylatklebstoffe sowie auch strahlenhärtende Klebstoffe. Die Dicke des Klebstoffauftrages wird bei der Klebstoffapplikation so gewählt, dass die Garne des Gewirkes fest im Klebstoff verankert sind.

Der Klebstoff zur Verbindung des Trägers mit dem Gewirke wird zweckmäßig in einem Muster appliziert, welches sich aus Klebeflächen und klebstofffreien Bereichen zusammensetzt. Vorzugsweise bildet der Klebstoff ein Muster aus parallelen Streifen, die quer zur bevorzugten Dehnungsrichtung des Trägers ausgerichtet sind. Da der Klebstoff nicht als zusammenhängender Film appliziert wird und die Klebeflächen keine wesentliche Ausdehnung in der bevorzugten Dehnungsrichtung aufweisen, sondern sich vorzugsweise als Streifen quer zur Dehnungsrichtung erstrecken, wird der Klebstoff bei einer Dehnung des Verbundstoffes keiner wesentlichen Beanspruchung ausgesetzt und behindert nicht die Ausdehnung des Gewirkes und des elastischen Trägers.

Der elastische Träger besteht vorzugsweise aus einer ein- oder mehrschichtigen perforierten Folie, wobei die Folie als Coextrudat eine elastomere Kernschicht und nichtelastomere Deckschichten, z. B. polyolefine Deckschichten, aufweisen kann. Die Perforation der Folie besteht aus 0,5 bis 2 mm großen Luftöffnungen, die mittels heißer Nadelwalzen erzeugt werden können.

Im Rahmen der Erfindung liegt es auch, dass der elastische Träger eine an das Gewirke angrenzende Schicht aus einem elastischen Spinnvlies aufweist, welches in der Dehnungsrichtung des Trägers bevorzugt dehnbar ist, und aus Filamenten besteht, die einen Filamentkem aus einem thermoplastischen Elastomer und einen durch Dehnung des Spinnvlieses verstreckten Filamentmantel aus einem nichtelastischen thermoplastischen Polymer aufweisen. Durch Verwendung eines elastischen Trägers, der als textiles Material ausgebildet ist oder zumindest eine an das Gewirke angrenzende Schicht aus einem textilen Material aufweist, kann der textile Charakter des Verbundstoffes noch verstärkt werden. Des Weiteren zeichnet sich das elastische Spinnvlies als Trägermaterial dadurch aus, dass es nach Maßgabe der Vordehnung der Filamente mit einer geringen Kraft bis zu einer deutlich wahrnehmbaren Dehngrenze gedehnt werden kann. Bei Erreichen der durch das Material festgelegten Dehngrenze ist ein starker Anstieg der zur weiteren Verdehnung nötigen Kraft zu bemerken, so dass eine Überdehnung des Verbundstoffes durch unsachgemäßen Gebrauch weitgehend ausgeschlossen ist. Ähnliche Eigenschaften stellen sich ein, wenn der elastische Träger aus einem monoaxial elastischen Verbundstoff besteht, der eine perforierte elastische Trägerfolie aus einem thermoplastischen Elastomer sowie ein- oder beidseitig aufkaschierte Schichten aus einem elastischen Spinnvlies aufweist.

Das Gewirke kann aus Monofilamentgamen oder Multifilamentgarnen gefertigt sein, wobei die Filamentgame beispielsweise aus einem Polyamid, Polyester, Polypropylen, Wolle oder Baumwolle bestehen können.

Gemäß einer bevorzugten Ausführung der Erfindung ist auf beiden Seiten des elastischen Trägers eine aus einem Gewirke bestehende Außenschicht angeordnet, wobei als Gewirke eine Kettwirkware bevorzugt ist, die in Dehnungsrichtung des Verbundstoffes leicht dehnbar ist und quer dazu einen großen Dehnungswiderstand aufweist.

Der elastische Träger kann eine geringere Länge aufweisen als die Außenschichten. Die Außenschichten sind in diesem Fall an ihren überstehenden Rändern unmittelbar miteinander verbunden. In den überstehenden Bereichen kann gegebenenfalls auch eine nichtelastische Folie einkaschiert sein.

Das außenseitige Gewirke des erfindungsgemäßen Verbundstoffes weist vorzugsweise ein Flächengewicht zwischen 15 und 50 g/m² auf. Die Schichtdicke des elastischen flächigen Trägers liegt vorzugsweise in einem Bereich zwischen 20 und 100 µm.

Der erfindungsgemäße Verbundstoff lässt sich in der bevorzugten Dehnungsrichtung um 50 % bis 150 % elastisch dehnen, wobei die irreversible Verformung nach mehreren Entlastungszyklen max. 15 % beträgt. Die für eine Dehnung um 50 % erforderliche Dehnungskraft bezogen auf eine Probenbreite von 50 mm beträgt vorzugsweise zwischen 2 bis 4 N/50 mm und die für eine Dehnung um 100 % erforderliche Dehnungskraft 5 bis 8 N/50 mm. Ferner zeichnet sich der erfindungsgemäße Verbundstoff durch eine hohe Reißfestigkeit von mehr als 25 N/50 mm in der bevorzugten Dehnungsrichtung aus.

Der erfindungsgemäße Verbundstoff eignet sich insbesondere zur Herstellung von elastischen Windelverschlüssen, die in Form von anatomisch geeigneten Seitenteilen mit Verschlusshaken versehen in Babywindeln und Inkontinenz produkten für Erwachsene eingesetzt werden. Die weiche Textiloberfläche der Kettenwirkware sorgt dabei für einen angenehmen Kontakt zur Haut.

Im Folgenden wird die Erfindung anhand einer lediglich ein Ausführungsbeispiel darstellenden Zeichnung erläutert. Es zeigen schematisch:
- **Fig. 1**: eine Draufsicht des erfindungsgemäßen Verbundstoffes,
- **Fig. 2**: das als Außenschicht des Verbundstoffes verwendete Gewirke in einer gegenüber Fig. 1 vergrößerten Darstellung,
- **Fig. 3**: ein Verfahren zur Herstellung des in Fig. 1 dargestellten Verbund- stoffes.

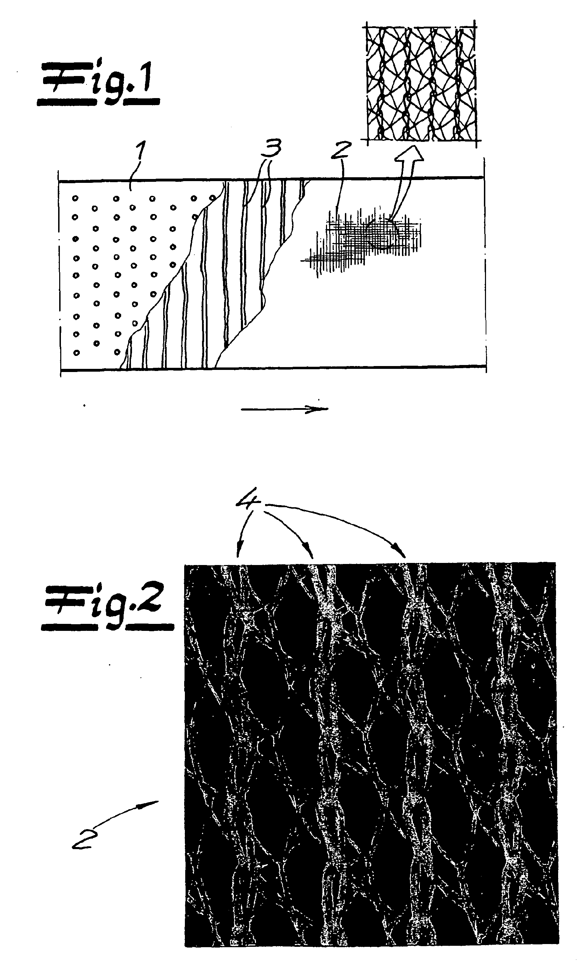

Der in Fig. 1 dargestellte Verbundstoff ist elastisch und luftdurchlässig und eignet sich zur Fertigung von elastischen Windelverschlussbändern und von elastischen Seitenteilen an Windeln. Er weist einen elastischen Träger 1 in Form einer perforierten Folie auf, die in einer Richtung bevorzugt dehnbar ist. Die bevorzugte Dehnungsrichtung ist in Fig. 1 mit einem Pfeil gekennzeichnet. Auf beiden Seiten des Trägers 1 ist eine textile Außenschicht aufkaschiert, die aus einem Gewirke 2 besteht. Das Gewirke 2 ist mit der elastischen Folie 1 verklebt, wobei der Klebstoff in einem Muster appliziert ist, welches sich aus Klebeflächen und klebstofffreien Bereichen zusammensetzt. Im Ausführungsbeispiel und gemäß einer bevorzugten Ausführung der Erfindung bildet der Klebstoff ein Muster aus parallelen Streifen 3, die quer zur bevorzugten Dehnungsrichtung des Trägers 1 ausgerichtet sind.

Bei dem Gewirke 2 handelt es sich um eine Kettwirkware mit gewirkten Ketten 4. Die Kettwirkware ist in Kettrichtung nur geringfügig dehnbar und weist quer dazu ein hohes Dehnungsvermögen auf. Die Kettwirkware 2 ist aus Monofilamentgam oder Multifilamentgarn gefertigt, wobei die Filamentgame aus Polyamid, Polyester, Polypropylen, Baumwolle, Wolle oder anderen textiltechnisch verarbeitbaren Materialien bestehen können. Die Kettwirkware 2 weist ein Flächengewicht zwischen 15 und 50 g/m² auf. Die elastische Trägerfolie 1 weist zweckmäßig eine Schichtdicke zwischen 20 µm und 100 µm auf. Geeignet sind insbesondere mehrschichtige Coextrusionsfolien, die einen elastischen Kern aus einem thermoplastischen Elastomer und polyolefinische Deckschichten aufweisen.

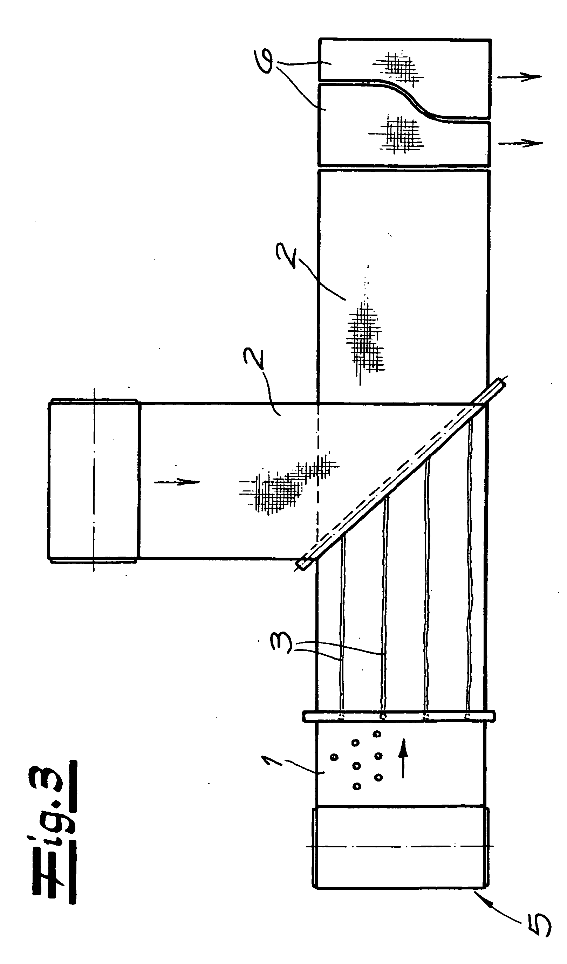

Sowohl die elastische Trägerfolie 1 als auch die als Außenschicht verwendete Kettwirkware 2 weisen eine bevorzugte Dehnungsrichtung auf und sind in der Querrichtung zugfest. Das erlaubt eine Fertigung nach dem in Fig. 3 dargestellten Kaschierverfahren. Auf die elastische Trägerfolie 1, die von einer Rolle 5 abgezogen wird, wird Klebstoff einseitig oder beidseitig in Form von Streifen 3 aufgetragen. Anschließend wird ein- oder beidseitig Kettwirkware 2 aufkaschiert, die in der in Fig. 3 dargestellten Maschinenrichtung (MD) zugfest ist. Von dem Kaschierverbund werden schließlich elastische Seitenteile 6 für Windeln ausgestanzt, die quer zur Maschinenlaufrichtung elastisch dehnbar sind.

## Patentansprüche

1. Monoaxial dehnbarer elastischer Verbundstoff, insbesondere zur Fertigung von elastischen Seitenteilen an Windeln und Windelverschlussbändern, mit
einem elastischen Träger (1), der in einer Richtung bevorzugt dehnbar ist, und
mindestens einer textilen Außenschicht, die aus einem Gewirke (2) besteht,
wobei der elastische Träger (1) aus einem flächigen Material besteht, welches eine bevorzugte Dehnungsrichtung aufweist und mit dem Gewirke (2) verklebt ist, **dadurch gekennzeichnet, dass** das Gewirke (2) aus einer Kettwirkware besteht, die in der Dehnungsrichtung des Verbundstoffes ein hohes Dehnungsvermögen aufweist und in der Querrichtung dazu steif ist.

2. Verbundstoff nach Anspruch 1, **dadurch gekennzeichnet, dass** der Klebstoff zur Verbindung des Trägers mit dem Gewirke in einem Muster appliziert ist, welches sich aus Klebeflächen und klebstofffreien Bereichen zusammensetzt.

3. Verbundstoff nach Anspruch 2, **dadurch gekennzeichnet, dass** der Klebstoff ein Muster aus parallelen Streifen (3) bildet, die quer zur bevorzugten Dehnungsrichtung des Trägers (1) ausgerichtet sind.

4. Verbundstoff nach einem der Ansprüche 1 bis 3, **dadurch gekennzeichnet, dass** der elastische Träger (1) luftdurchlässig ist.

5. Verbundstoff nach Anspruch 4, **dadurch gekennzeichnet, dass** der elastische Träger (1) aus einer ein- oder mehrschichtigen perforierten Folie besteht.

6. Verbundstoff nach einem der Ansprüche 1 bis 5, **dadurch gekennzeichnet, dass** der elastische Träger aus einer mehrschichtigen Coextrusionsfolie besteht, die eine elastomere Kernschicht und polyolefinische Deckschichten aufweist.

7. Verbundstoff nach einem der Ansprüche 1 bis 5, **dadurch gekennzeichnet, dass** der elastische Träger (1) eine an das Gewirke (2) angrenzende Schicht aus einem elastischen Spinnvlies aufweist, welches in der Dehnungsrichtung des Trägers bevorzugt dehnbar ist, und dass das Spinnvlies aus Filamenten besteht, die einen Filamentkem aus einem thermoplastischen Elastomer und einen durch Dehnung des Spinnvlieses verstreckten Filamentmantel aus einem nichtelastischen thermoplastischen Polymer aufweisen.

8. Verbundstoff nach einem der Ansprüche 1 bis 7, **dadurch gekennzeichnet, dass** der elastische Träger (1) aus einem monoaxial elastischen Verbundstoff besteht, der eine perforierte elastische Trägerfolie aus einem thermoplastischen Elastomer sowie ein- oder beidseitig aufkaschierte Schichten aus einem elastischen Spinnvlies aufweist.

9. Verbundstoff nach einem der Ansprüche 1 bis 8, **dadurch gekennzeichnet, dass** das Gewirke (2) aus Monofilamentgarnen oder Multifilamentgarnen gefertigt ist, wobei die Filamentgarne aus einem Polyamid, Polyester, Polypropylen, Wolle oder Baumwolle bestehen.

10. Verbundstoff nach einem der Ansprüche 1 bis 9, **dadurch gekennzeichnet, dass** auf beiden Seiten des elastischen Trägers (1) eine aus einem Gewirke (2) bestehende Außenschicht angeordnet ist, wobei das Gewirke (2) jeweils einer Kettwirkware besteht, die in der Dehnungsrichtung des Verbundstoffes leicht dehnbar ist und quer dazu einen großen Dehnungswiderstand aufweist.

11. Verbundstoff nach Anspruch 10, **dadurch gekennzeichnet, dass** der elastische Träger (1) eine geringere Länge aufweist als die Außenschichten und dass die Außenschichten an ihren überstehenden Rändern unmittelbar miteinander verbunden sind.

12. Verbundstoff nach einem der Ansprüche 1 bis 11, **dadurch gekennzeichnet, dass** das Gewirke (2) ein Flächengewicht von 15 bis 50 g/m² und der elastische flächige Träger (1) eine Schichtdicke von 20 bis 100 µm aufweisen.

13. Verbundstoff nach einem der Ansprüche 1 bis 12, **dadurch gekennzeichnet, dass** der Verbundstoff in der bevorzugten Dehnungsrichtung um 50 % bis 150 % elastisch dehnbar ist, eine verbleibende Verformung nach mehreren Entlastungszyklen von maximal 15 % sowie eine hohe Reißfestigkeit in der bevorzugten Dehnungsrichtung von mehr als 25 N bezogen auf eine Probenbreite von 50 mm aufweist.

## Claims

1. A monoaxially extensible elastic composite material, in particular for the production of elastic sides on nappies and nappy sealing tapes, with
an elastic support (1), which is preferably extensible in one direction, and
at least one textile outer layer, which consists of a knitted fabric (2),
wherein the elastic support (1) consists of a sheet material, which has a preferred direction of extension and is adhesively bonded with the knitted fabric (2), **characterised in that** the knitted fabric (2) consists of a warp knit fabric, which has a high extensibility in the direction of extension of the composite material and is rigid in the direction transverse to the latter.

2. The composite material in accordance with Claim 1, **characterised in that** the adhesive for purposes of connecting the support with the knitted fabric is applied in a pattern, which is composed of surface areas of adhesive and areas that are free of adhesive.

3. The composite material in accordance with Claim 2, **characterised in that** the adhesive forms a pattern of parallel strips (3), which are aligned transverse to the preferred direction of extension of the support (1).

4. The composite material in accordance with one of the Claims 1 to 3, **characterised in that** the elastic support (1) is permeable to air.

5. The composite material in accordance with Claim 4, **characterised in that** the elastic support (1) consists of a single-layer or multi-layer perforated film.

6. The composite material in accordance with one of the Claims 1 to 5, **characterised in that** the elastic support consists of a multi-layer coextrusion film, which has an elastomer core layer, and polyolefin cover layers.

7. The composite material in accordance with one of the Claims 1 to 5, **characterised in that** the elastic support (1) has a layer adjacent to the knitted fabric (2) of an elastic spunbond, which is preferably extensible in the direction of extension of the support, and **in that** the spunbond consists of filaments, which have a filament core of a thermoplastic elastomer and a filament sheath of a non-elastic thermoplastic polymer that is stretched by extension of the spunbond.

8. The composite material in accordance with one of the Claims 1 to 7, **characterised in that** the elastic support (1) consists of a monoaxial elastic composite material, which has a perforated elastic support film of a thermoplastic elastomer, and also layers of an elastic spunbond that are laminated on one or both sides.

9. The composite material in accordance with one of the Claims 1 to 8, **characterised in that** the knitted fabric (2) is produced from mono-filament yarns or multi-filament yarns, wherein the filament yarns consist of a polyamide, polyester, polypropylene, wool, or cotton.

10. The composite material in accordance with one of the Claims 1 to 9, **characterised in that** a knitted fabric (2) is arranged on both sides of the elastic support (1) as outer layer, wherein the knitted fabric (2) in each case consists of a warp knit fabric, which is easily extensible in the direction of extension of the composite material, and has a high resistance to extension transverse to the latter.

11. The composite material in accordance with Claim 10, **characterised in that** the elastic support (1) has a lesser length than the outer layers, and **in that** the outer layers are directly connected with one another on their projecting edges.

12. The composite material in accordance with one of the Claims 1 to 11, **characterised in that** the knitted fabric (2) has a mass per unit area of 15 to 50 g/m² and the elastic planar support (1) has a layer thickness of 20 to 100 µm.

13. The composite material in accordance with one of the Claims 1 to 12, **characterised in that** the composite material can be elastically extended by 50 % to 150 % in the preferred direction of extension, has a residual deformation after a plurality of unloading cycles of a maximum of 15 %, and has a high resistance to tearing in the preferred direction of extension of more than 25 N with reference to a sample width of 50 mm.

## Revendications

1. Matériau composite élastique étirable monoaxialement, destiné notamment à la fabrication de pièces latérales extensibles de couches et bandes de fermeture de couches, comportant
un support élastique (1) qui est de préférence extensible dans un sens et
au moins une couche textile extérieure qui est composée de maille (2),
le support élastique (1) consistant en un matériau plat qui présente un sens d'extension préférentiel et est collé à la maille (2), **caractérisé en ce que** la maille (2) consiste en un tricot en chaîne qui présente une grande capacité d'étirement dans le sens d'étirement du matériau composite et qui est rigide dans le sens transversal par rapport à celui-ci.

2. Matériau composite selon la revendication 1, **caractérisé en ce que** la colle permettant de raccorder le support à la maille est appliquée suivant un motif qui est composé de surfaces encollées et de parties non encollées.

3. Matériau composite selon la revendication 2, **caractérisé en ce que** la colle forme un motif de bandes parallèles (3) qui sont orientées transversalement par rapport au sens d'étirement préférentiel du support (1).

4. Matériau composite selon une des revendications 1 à 3, **caractérisé en ce que** le support élastique (1) laisse passer l'air.

5. Matériau composite selon la revendication 4, **caractérisé en ce que** le support élastique (1) est composé d'un film perforé mono- ou multicouche.

6. Matériau composite selon une des revendications 1 à 5, **caractérisé en ce que** le support élastique (1) est composé d'une surface coextrudée multicouche qui présente une couche centrale en élastomère et des couches de couverture polyoléfiniques.

7. Matériau composite selon une des revendications 1 à 5, **caractérisé en ce que** le support élastique (1) présente une couche jouxtant la maille (2) et composée d'un non-tissé élastique qui est de préférence étirable dans le sens d'étirement du support et que le non-tissé est composé de filaments qui présentent un noyau filamenteux en élastomère thermoplastique et une enveloppe filamenteuse étendue étirement du non-tissé et faite de polymère thermoplastique non élastique.

8. Matériau composite selon une des revendications 1 à 7, **caractérisé en ce que** le support élastique (1) consiste en un matériau composite élastique monoaxialement et qui présente un film porteur élastique perforé en élastomère thermoplastique et des couches plaquées dessus uni- ou bilatéralement en non-tissé élastique.

9. Matériau composite selon une des revendications 1 à 8, **caractérisé en ce que** la maille (2) est fabriquée à partir de fils monofilaments ou de fils multifilaments, les filaments étant composés de polyamide, polyester, polypropopylène, laine ou coton.

10. Matériau composite selon une des revendications 1 à 9, **caractérisé en ce que**, sur les deux faces du support élastique (1), une couche extérieure composée de maille (2) est disposée, la maille (2) consistant respectivement en un tricot en chaîne qui est légèrement étirable dans le sens d'étirement du matériau composite et présente une grande résistance à l'étirement transversalement à ce sens.

11. Matériau composite selon la revendication 10, **caractérisé en ce que** le support élastique (1) présente une longueur plus réduite que les couches extérieures et que les couches extérieures sont reliées directement entre elles au niveau de leurs bords surélevés.

12. Matériau composite selon une des revendications 1 à 11, **caractérisé en ce que** la maille (2) présente un grammage de 15 à 50 g/m² et le support élastique plat (1) une épaisseur de couche de 20 à 100 µm.

13. Matériau composite selon une des revendications 1 à 12, **caractérisé en ce que** le matériau composite est étirable élastiquement de 50 % à 150 % dans le sens d'étirement préférentiel et présente une déformation résiduelle au bout de plusieurs cycles de délestage de 15 % au maximum ainsi qu'une résistance à la rupture élevée de plus de 25 N en référence à une largeur d'échantillon de 50 mm dans le sens d'étirement préférentiel.
